(19) Europäisches Patentamt
European Patent Office
Office européen des brevets

(11) EP 4 691 508 A1

(12) **EUROPEAN PATENT APPLICATION**
published in accordance with Art. 153(4) EPC

(43) Date of publication:
**11.02.2026 Bulletin 2026/07**

(21) Application number: **24780258.0**

(22) Date of filing: **26.03.2024**

(51) International Patent Classification (IPC):
***A61L 33/06*** *(2006.01)* ***A61L 29/06*** *(2006.01)*

(52) Cooperative Patent Classification (CPC):
**A61L 29/06; A61L 33/06**

(86) International application number:
**PCT/JP2024/011857**

(87) International publication number:
**WO 2024/204145 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.03.2023 JP 2023053423**

(71) Applicant: **Toyobo Co., Ltd.**
**Kita-ku**
**Osaka-shi, Osaka 530-0001 (JP)**

(72) Inventors:
- **AHAMED, Kashfia**
  **Otsu-shi, Shiga 520-0292 (JP)**
- **KAWAKATSU, Yuta**
  **Otsu-shi, Shiga 520-0292 (JP)**
- **NAKAYAMA, Shohei**
  **Otsu-shi, Shiga 520-0292 (JP)**

(74) Representative: **Vossius & Partner Patentanwälte Rechtsanwälte mbB**
**Siebertstrasse 3**
**81675 München (DE)**

(54) **METHOD FOR MANUFACTURING ANTITHROMBOTIC MEDICAL DEVICE**

(57) The objective of the present invention is to provide a method for coating a polyurethane medical device such as a catheter with an antithrombotic material for antithromboticity with suppressing dimensional change such as swelling and shrinkage of the medical device. The method for producing an antithrombotic medical device according to the present invention is characterized in comprising the steps of dissolving an antithrombotic material comprising a (meth)acrylate copolymer in an aliphatic hydrocarbon solvent to prepare an aliphatic hydrocarbon solution, coating a medical device with the aliphatic hydrocarbon solution, and removing the aliphatic hydrocarbon solvent from a surface of the medical device, wherein the medical device is composed of polyurethane.

[Fig. 2]

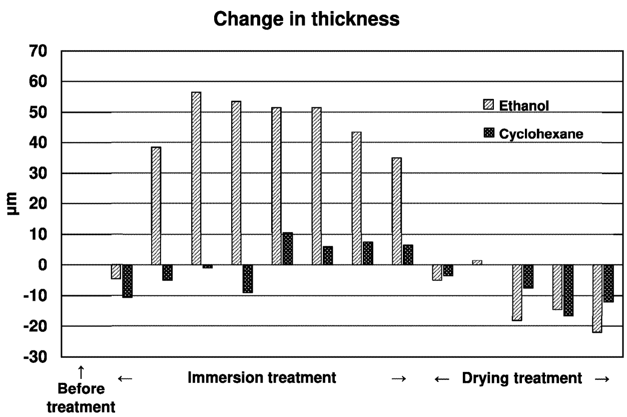


EP 4 691 508 A1

Processed by Luminess, 75001 PARIS (FR)

## Description

### TECHNICAL FIELD

The present invention relates to a technology to coat at least a part of a region of a medical device such as a catheter to be contacted with body fluid with an antithrombotic material comprising a (meth)acrylate copolymer.

### BACKGROUND ART

A catheter is sometimes indwelled in a body for diagnosis and treatment. An example of a catheter includes a catheter for central infusion, an intravenous indwelling needle, a catheter for epidural anesthesia, a catheter for monitoring cardiac output and a feeding catheter. A catheter is used for injecting a transfusion material such as a saline solution, an electrolyte, a nutritional supplement and a drug, blood and an anesthetic, for collecting blood, and for measuring a blood component, cardiac output, regional blood pressure and blood gas.

An example of a tube material for an indwelling vascular catheter includes silicone resin, vinyl chloride resin, nylon, polyolefin and ethylene-tetrafluoroethylene. When such a catheter is indwelled in a body, an inflammatory reaction may be caused to produce active oxygen and a hydrolytic enzyme in some cases. As a result, many of the above-described materials are unsuitable for the indwelling in a body for a long time, since the materials are influenced by hydrolysis and active oxygen in a living body and thus the physical property thereof is easily deteriorated. When a medical device such as a catheter is used with being contacted with blood, 3 items of (a) suppression of a blood coagulation system, (b) suppression of adhesion and activation of blood platelet, and (c) suppression of complement activation are important for biocompatibility.

Patent document 1 discloses an antithrombotic material produced from an alkyl (meth)acrylate, a silicone (meth) acrylate and a methoxypolyethylene glycol (meth)acrylate, and a suspension for treating a medical material prepared by dispersing the antithrombotic material in a mixed solvent having a weight ratio of water and an organic solvent such as ethanol and isopropyl alcohol of 3-30/97-70 in a concentration of 0.001 to 10 weight% for improving biocompatibility.

Patent document 2 discloses a technology to coat the surface of a catheter with an antithrombotic material containing a (meth)acrylate copolymer with using ethanol or a mixed solvent ethanol and water.

But when a solvent such as ethanol is used for coating the surface of a catheter, especially a polyurethane resin catheter, with an antithrombotic material containing a (meth)acrylate copolymer, there is a problem that the polyurethane resin shrinks or swells and thus the quality is deteriorated. For example, the dimensions are not stable among the products.

### PRIOR ART DOCUMENT

### PATENT DOCUMENT

Patent document 1: JP 4100452 B
Patent document 2: JP 2009-261437 A

### DISCLOSURE OF THE INVENTION

### PROBLEMS TO BE SOLVED BY THE INVENTION

The objective of the present invention is to provide a method for coating a polyurethane medical device such as a catheter with an antithrombotic material for antithromboticity with suppressing dimensional change such as swelling and shrinkage of the medical device.

### MEANS FOR SOLVING THE PROBLEMS

The inventors of the present invention completed the present invention by finding that the dimensional change of an antithrombotic medical device produced by applying a solution prepared by dissolving an antithrombotic material in an aliphatic hydrocarbon solvent on a polyurethane medical device due to the application of the solution is less. The features of the present invention are hereinafter described.

[1] A method for producing an antithrombotic medical device, comprising the steps of:

dissolving an antithrombotic material comprising a (meth)acrylate copolymer in an aliphatic hydrocarbon solvent to prepare an aliphatic hydrocarbon solution,
coating a medical device with the aliphatic hydrocarbon solution, and
removing the aliphatic hydrocarbon solvent from a surface of the medical device,
wherein the medical device is composed of polyurethane.

[2] The method according to the above [1], wherein a concentration of the (meth)acrylate copolymer in the aliphatic hydrocarbon solution is 0.01 mass% or more and 10 mass% or less.
[3] The method according to the above [1] or [2], wherein a temperature of the aliphatic hydrocarbon solution is 10°C or higher and 45°C or lower.
[4] The method according to any one of the above [1] to [3], wherein the aliphatic hydrocarbon solvent is removed by blowing air on the medical device in a flow rate of 1 mL/min or more and 100 L/min or less.
[5] The method according to the above [4], wherein the air is blown on the medical device for 1 second or more and 180 seconds or less.
[6] The method according to any one of the above [1] to [5], wherein the (meth)acrylate copolymer comprises an alkyl (meth)acrylate unit represented by the following formula (I), a silicone (meth)acrylate unit represented by the following formula (II) and a methoxypolyethylene glycol (meth)acrylate unit represented by the following formula (III):

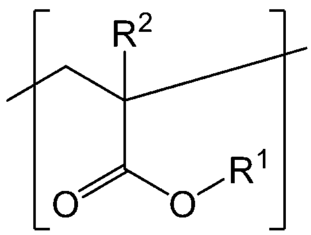

(I)

wherein $R^1$ is a $C_{6-20}$ alkyl group, and $R^2$ is a hydrogen atom or a methyl group,

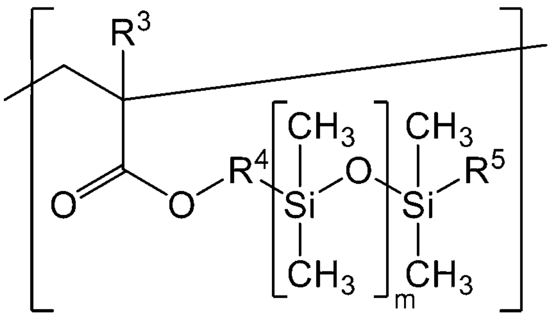

(II)

wherein $R^3$ is a hydrogen atom or a methyl group, $R^4$ is a $C_{1-6}$ alkylene group, $R^5$ is a $C_{1-6}$ alkyl group, and m is an integer of 1 or more and 30 or less,

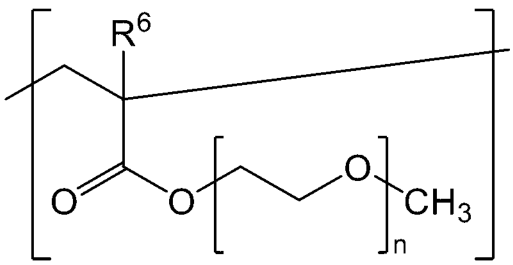

(III)

wherein $R^6$ is a hydrogen atom or a methyl group, and n is an integer of 2 or more and 4 or less.

[7] The method according to the above [6], wherein a molar ratio of the alkyl (meth)acrylate unit/the silicone (meth) acrylate unit/the methoxypolyethylene glycol (meth)acrylate unit in the (meth)acrylate copolymer is 80-20/10-0.01/10-79.99.
[8] The method according to any one of the above [1] to [7], wherein a weight average molecular weight of the (meth)acrylate copolymer is 50,000 or more and 1,500,000 or less.

[9] The method according to any one of the above [1] to [8], wherein a reduced viscosity of the (meth)acrylate copolymer is 0.18 dl/g or more and 0.50 dl/g or less.

[10] The method according to any one of the above [1] to [9], wherein the medical device is coated with 0.005 mg/$cm^2$ or more and 0.06 mg/$cm^2$ or less of the (meth)acrylate copolymer.

[11] A medical device,

composed of polyurethane, and
coated with 0.005 mg/$cm^2$ or more and 0.06 mg/$cm^2$ or less of a (meth)acrylate copolymer.

EFFECT OF THE INVENTION

Not only at least a part of the part to be contacted with a body fluid of a polyurethane medical device can be uniformly coated with an antithrombotic material but also the swelling and the shrinkage caused by a coating treatment using an organic solvent can be suppressed according to the present invention. Thus, a polyurethane medical device having excellent antithromboticity can be provided by the present invention.

BRIEF DESCRIPTION OF THE DRAWINGS

[Figure 1] A conceptual diagram for explaining the effect of a coating solvent on a tube as a polyurethane medical device

[Figure 2] A graph to demonstrate the measurement result of the effect of a coating solvent on a tube thickness

[Figure 3] A graph to demonstrate the measurement result of the effect of a coating solvent on a tube internal diameter

[Figure 4] A graph to demonstrate the measurement result of a coating solvent on a tube external diameter

[Figure 5] Photographs to demonstrate the result of blood compatibility test using the tubes of Example

MODE FOR CARRYING OUT THE INVENTION

The method for producing an antithrombotic medical device according to the present invention is characterized in comprising the steps of dissolving an antithrombotic material comprising a (meth)acrylate copolymer in an aliphatic hydrocarbon solvent to prepare an aliphatic hydrocarbon solution, coating a medical device with the aliphatic hydrocarbon solution, and removing the aliphatic hydrocarbon solvent from a surface of the medical device, wherein the medical device is composed of polyurethane. The present invention is hereinafter described but is not restricted to the following specific embodiments.

1. Step to prepare solution

The antithrombotic material comprising a (meth)acrylate copolymer is dissolved in an aliphatic hydrocarbon solvent to prepare an aliphatic hydrocarbon solution in this step.

The (meth)acrylate copolymer comprises a hydrophobic (meth)acrylate unit and a hydrophilic (meth)acrylate unit, and is resistant to the contact with blood in the present invention. The term "resistant to the contact with blood" means that when the (meth)acrylate copolymer is immersed in an alcohol immersion treatment liquid at room temperature for 16 hours, a predetermined or more amount of the (meth)acrylate copolymer is not dissolved to remain and show antithromboticity in this disclosure. When 0.1 $\mu$g/$cm^2$ or more of the (meth)acrylate copolymer remains in the case where the (meth)acrylate copolymer is immersed in an alcohol immersion treatment liquid at room temperature for 16 hours, it can be judged that the (meth)acrylate copolymer has sufficient antithromboticity even in the case where the (meth)acrylate copolymer is contacted with blood at 37°C for 30 days. A mixed solvent of methanol/ethanol = 80/20 by mass is preferred as an alcohol immersion treatment liquid, since the elution ability of the mixed solvent is stronger than that of blood. The (meth)acrylate copolymer of the present invention is preferably a viscous liquid at room temperature. When the (meth) acrylate copolymer which is a viscous liquid at room temperature is used for coating a medical device or the like, the elution thereof into blood can be suppressed.

The hydrophobic (meth)acrylate unit may comprise the other hydrophobic (meth)acrylate unit in addition to the silicone (meth)acrylate unit (II) in the present invention. The other hydrophobic (meth)acrylate unit is not particularly restricted, and an example thereof preferably includes the alkyl (meth)acrylate unit represented by the following formula (I). The carbon number of $R^1$ is preferably 6 or more and 20 or less and more preferably 8 or more and 12 or less in the following formula (I). An example of $R^1$ includes n-hexyl, n-heptyl, n-octyl, 2-ethylhexyl, n-nonyl, n-decyl, n-lauryl, n-tetradecyl, n-hexadecyl, n-octadecyl and n-icosyl, and $R^1$ is preferably 2-ethylhexyl and n-lauryl in terms of a cost and performance.

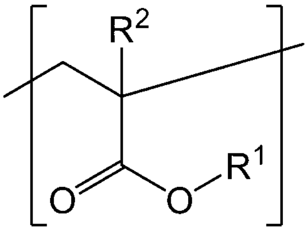

(I)

wherein $R^1$ is a $C_{6-20}$ alkyl group and $R^2$ is a hydrogen atom or a methyl group.

The hydrophobic (meth)acrylate unit preferably comprises the silicone (meth)acrylate unit represented by the following formula (II) in the present invention. The silicone (meth)acrylate unit is preferably a silicone (meth)acrylate unit of which repetition number "m" of the dimethylsiloxane is 1 or more and 30 or less. When the repetition number "m" is included in the above-described range, the copolymer may be retained on the surface of a medical device more surely and the handling property thereof is excellent due to the appropriate viscosity of the copolymer. The repetition number "m" is preferably 1 or more and 20 or less.

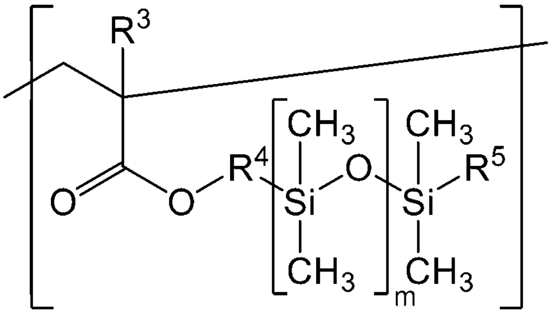

(II)

wherein $R^3$ is a hydrogen atom or a methyl group, $R^4$ is a $C_{1-6}$ alkylene group, $R^5$ is a $C_{1-6}$ alkyl group, and m is an integer of 1 or more and 30 or less.

The hydrophilic (meth)acrylate unit preferably comprises the methoxypolyethylene glycol (meth)acrylate represented by the following formula (III) in the present invention. The repetition number "n" of the ethylene oxide in the following formula (III) is preferably 2 or more and 10 or less. When the ethylene oxide repetition number "n" is 2 or more, the adhesion of platelet and the adsorption of a plasma protein can be suppressed more surely. When the "n" is 10 or less, the elution of the copolymer into blood can be suppressed more surely. An example of the specific ethylene oxide structure includes diethylene glycol structure, triethylene glycol structure, tetraethylene glycol structure, pentaethylene glycol structure, hexaethylene glycol structure, heptaethylene glycol structure, octaethylene glycol structure, nonaethylene glycol structure and decaethylene glycol structure. The ethylene oxide repetition number "n" is preferably 2 or more and 5 or less. The ethylene oxide structure is more preferably tetraethylene glycol (meth)acrylate structure of which repetition number is 4 and triethylene glycol (meth)acrylate structure of which repetition number is 3, and particularly preferably triethylene glycol (meth)acrylate structure of which repetition number is 3.

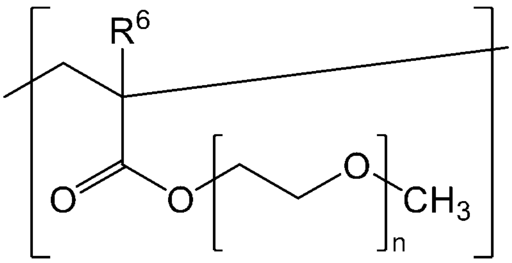

(III)

wherein $R^6$ is a hydrogen atom or a methyl group and "n" is an integer of 2 or more and 4 or less.

An example of the water-insoluble (meth)acrylate copolymer of the present invention includes silicone (meth) acrylate-methoxydiethylene glycol (meth)acrylate copolymer, silicone (meth)acrylate-methoxytriethylene glycol (meth) acrylate copolymer, silicone (meth)acrylate-methoxytetraethylene glycol (meth)acrylate copolymer, silicone (meth)acrylate-n-hexyl (meth)acrylate-methoxydiethylene glycol (meth)acrylate copolymer, silicone (meth)acrylate-n-hexyl (meth) acrylate-methoxytriethylene glycol (meth)acrylate copolymer, silicone (meth)acrylate-n-hexyl (meth)acrylate-methoxytetraethylene glycol (meth)acrylate copolymer, silicone (meth)acrylate-n-octyl (meth)acrylate-methoxydiethylene glycol (meth)acrylate copolymer, silicone (meth)acrylate-n-octyl (meth)acrylate-methoxytriethylene glycol (meth)acrylate copolymer, silicone (meth)acrylate-n-octyl (meth)acrylate-methoxytetraethylene glycol (meth)acrylate copolymer, silicone (meth)acrylate-2-ethylhexyl (meth)acrylate-methoxydiethylene glycol (meth)acrylate copolymer, silicone (meth)acrylate-2-ethylhexyl (meth)acrylate-methoxytriethylene glycol (meth)acrylate copolymer, silicone (meth)acrylate-2-ethylhexyl (meth)acrylate-methoxytetraethylene glycol (meth)acrylate copolymer, silicone (meth)acrylate-lauryl (meth)acrylate-methoxydiethylene glycol (meth)acrylate copolymer, silicone (meth)acrylate-lauryl (meth)acrylate-methoxytriethylene glycol (meth)acrylate copolymer, silicone (meth)acrylate-lauryl (meth)acrylate-methoxytetraethylene glycol (meth) acrylate copolymer, silicone (meth)acrylate-n-nonyl (meth)acrylate-methoxydiethylene glycol (meth)acrylate copolymer, silicone (meth)acrylate-n-nonyl (meth)acrylate-methoxytriethylene glycol (meth)acrylate copolymer, silicone (meth)acrylate-n-nonyl (meth)acrylate-methoxytetraethylene glycol (meth)acrylate copolymer, silicone (meth)acrylate-n-decyl (meth)acrylate-methoxydiethylene glycol (meth)acrylate copolymer, silicone (meth)acrylate-n-decyl (meth)acrylate-methoxytriethylene glycol (meth)acrylate copolymer, silicone (meth)acrylate-n-decyl (meth)acrylate-methoxytetraethylene glycol (meth)acrylate copolymer, silicone (meth)acrylate-lauryl (meth)acrylate-methoxydiethylene glycol (meth) acrylate copolymer, silicone (meth)acrylate-lauryl (meth)acrylate-methoxytriethylene glycol (meth)acrylate copolymer and silicone (meth)acrylate-lauryl (meth)acrylate-methoxytetraethylene glycol (meth)acrylate copolymer.

The molar ratio of the alkyl (meth)acrylate unit (I)/the silicone (meth)acrylate unit (II)/the methoxypolyethylene glycol (meth)acrylate unit (III) in the (meth)acrylate copolymer is not particularly restricted and may be adjusted to, for example, 80-20/10-0.01/10-79.99 on the proviso that the total of each of the unit is 100. When the hydrophobic (meth) acrylate unit, i.e. the total of the alkyl (meth)acrylate unit (I) and the silicone (meth)acrylate unit (II), is 20.01 mol% or more to the total, the copolymer is hardly dissolved in blood or the like. When the hydrophobic (meth)acrylate unit is 90 mol% or less, the copolymer is sufficiently suitable for blood. The above-described molar ratio is preferably 80-50/5-0.01/15-49.99, more preferably 77-55/5-0.01/18-44.99, and even more preferably 73-57/5-0.01/22-42.99.

The copolymer does not necessarily comprise the silicone (meth)acrylate unit as the hydrophobic (meth)acrylate unit in the present invention. Silicone has the advantage of exhibiting various stable properties over a wide temperature range due to excellent heat resistance, excellent cold resistance and low glass transition temperature (Tg) based on the basic skeleton thereof. In addition, silicone has the advantages of acid resistance, alkali resistance and high chemical stability due to large binding energy. Further, a silicone (meth)acrylate monomer is preferably used as the raw material of the (meth)acrylate copolymer according to the present invention, since the monomer has excellent copolymerizability with other (meth)acrylate monomers. Silicone (meth)acrylate is recognized as a material with high compatibility with a living body. For example, silicone (meth)acrylate has been used as a raw material for contact lenses in recent years. Thus, even if the content amount of a silicone (meth)acrylate in the antithrombotic material is excessively large, a problem may not be caused. The silicone (meth)acrylate may be a silicone (meth)acrylate-methoxypolyethylene glycol (meth)acrylate copolymer. It may be disadvantageous in terms of cost-effectiveness to use a silicone (meth)acrylate only as a hydrophobic (meth)acrylate, since the raw materials of a silicone (meth)acrylate are currently relatively expensive. The proportion of the silicone (meth)acrylate unit in the hydrophobic (meth)acrylate unit may be adjusted to, for example, 50 mass% or less with comprehensively considering performance, quality, a cost or the like. The proportion is preferably 40 mass% or less and more preferably 35 mass% or less. The silicone (meth)acrylate and the above-described alkyl (meth) acrylate may be mixed, and the mixture may be used. On the one hand, the proportion of the silicone (meth)acrylate unit in the hydrophobic (meth)acrylate unit is preferably 0.1 mass% or more. When the proportion is 0.1 mass% or more, hydrolysis hardly proceeds during the preservation of the copolymer and thus the antithrombotic material is excellent in long-term stability. The proportion is more preferably 0.5 mass% or more and even more preferably 1.0 mass% or more.

The (meth)acrylate copolymer may be a copolymer in which hydrophobic monomers and hydrophilic monomers are alternately arranged, but may also be a copolymer consisting of segments or blocks composed of hydrophobic monomers and segments or blocks composed of hydrophilic monomers in accordance with the analysis of the total amount. The segment or the block composed of the hydrophobic monomer may possibly have the function to fix the segment or the block composed of the hydrophilic monomer to form a complex structure such as socalled micro phase separation structure and a mosaic patternlike structure. Though the molecular weight of the copolymer and the type and the property of the hydrophilic monomer may also slightly have some effect, when the amount of the hydrophobic monomer is adjusted to be slightly large, the elution of the segment or the block composed of the hydrophilic monomer from the copolymer may be suppressed. In addition, when the amount of the hydrophobic segment or the hydrophobic block is adjusted to be somewhat large, the affinity of the copolymer for a hydrophobic medical device may be improved and the copolymer may play a useful role in adhering to a medical device as a coating layer. It is currently not possible to accurately verify whether or not the copolymer has such a segment or block and how the segment or the block plays a function in the hydrophilic condition on the basis of technical evidence, but the copolymer is a polymer material with good biocompatibility.

A methoxypolyethylene glycol (meth)acrylate homopolymer is excellent in blood compatibility due to high

hydrophilicity. On the one hand, when the homopolymer is contacted with blood or the like for a long time, the homopolymer has a problem that the homopolymer is gradually eluted due to the hydrophilicity. The inventors actively researched the material that is not only highly compatible with blood but also durable for long-term use. As a result, the inventors found that the copolymer having appropriate hydrophobicity for inhibiting the elution of the copolymer to blood or the like and flexibility for inhibiting the physical abrasion of the coating layer can dissolve the problem.

The copolymer in the coating liquid is substantially composed of two kinds of monomer components that serve two interface functions different from each other. The monomer components are a hydrophilic monomer, segment or block having an antithrombotic function and a function to inhibit the elution into blood and a hydrophobic monomer, segment or block having affinity for a medical device and a function to adhere on a medical device. In addition, the monomers, segments or blocks that constitute the copolymer complement each other in the molecular structure, and the molecule that is stable toward elution and dispersion may be bound to or form the structure.

An example of an index to express a molecular weight of a copolymer includes a number average molecular weight and a weight average molecular weight. Since weight average molecular weight has a greater effect on stability and adhesion, weight average molecular weight is selected as an index in the present invention. The weight average molecular weight of the copolymer is preferably 50,000 or more and 1,500,000 or less. When the weight average molecular weight is 50,000 or more, not only the elution into blood can be suppressed more surely but also the strength and the stability of the coating layer can be ensured more surely. In addition, since the viscosity of the coating solution is increased, the adherence of the copolymer on a medical device is also improved. The weight average molecular weight is preferably 60,000 or more. When the weight average molecular weight is 1,500,000 or less, the (meth)acrylate copolymer can be easily treated in the case where a medical device is coated with the (meth)acrylate copolymer. The weight average molecular weight is more preferably 1,000,000 or less and even more preferably 500,000 or less. A weight average molecular weight means the value calculated by dividing the sum of the molecular weights of all molecules by the sum of the weights of the molecules, and is one of the indexes of the property of a polymer.

It is a very important technological requirement to adjust the weight average molecular weight of the (meth) acrylate copolymer to 50,000 or more and 1,500,000 or less in order to solve the specific technical problems such as the purification of the copolymer, the treatment of the coating liquid, the adaptability to a medical device and the stability of the coating layer.

An example of a method for measuring the weight average molecular weight includes end group determination method, osmometry, vapor pressure osmometry, vapor pressure depression method, freezing point depression method, ebullioscopic method and gel permeation chromatography (GPC) method. A conventional gel permeation chromatography (GPC) method may be used in the present invention in terms of easy operation.

The reduced viscosity ($\eta sp/c$) of the (meth)acrylate copolymer is preferably 0.18 dl/g or more and 3.00 dl/g or less in the present invention. When the copolymer having the viscosity included in the range is used as an antithrombotic material, the copolymer is excellent in the adherence on a medical device and the antithromboticity can be maintained for a long time in the case where a medical device such as an artificial heart-lung circuit and a catheter is coated with the copolymer. The reduced viscosity is more preferably 0.18 dl/g or more and 1.50 dl/g or less and even more preferably 0.18 dl/g or more and 0.50 dl/g or less.

The (meth)acrylate copolymer is preferably soluble in an aliphatic hydrocarbon solvent in the present invention. It is more preferred that the (meth)acrylate copolymer is soluble in an aliphatic hydrocarbon solvent having the carbon number of 5 or more and 8 or less, since the (meth)acrylate copolymer can be easily dried after the coating. The term "soluble" means that when 1 g of the (meth)acrylate copolymer is immersed in 10 mL of the aliphatic hydrocarbon solvent at 25°C, at least 90 mass% of the (meth)acrylate copolymer is dissolved at room temperature for 16 hours or less.

The (meth)acrylate copolymer may be any one of a random copolymer, a block copolymer and a graft copolymer in the present invention. The copolymerization reaction to produce the (meth)acrylate copolymer is not particularly restricted, and a publicly known method such as radical polymerization, ion polymerization, photopolymerization and polymerization using a macromer may be used.

For example, radical polymerization may be started by dissolving each (meth)acrylate monomer in a solvent and adding a radical polymerization initiator thereto. A proportion of each structure unit in the (meth)acrylate copolymer may correspond to the use proportion of each (meth)acrylate monomer. In other words, the use proportion of each (meth) acrylate monomer may be adjusted depending on the proportion of each structure unit in the (meth)acrylate copolymer as the target compound. An example of the solvent includes an alcohol solvent such as methanol, ethanol and 2-propanol; water; an aromatic hydrocarbon solvent such as toluene and xylene; an aliphatic hydrocarbon solvent such as n-hexane and n-heptane; an ester solvent such as ethyl acetate and butyl acetate; a ketone solvent such as acetone, methyl ethyl ketone and methyl isobutyl ketone; and a mixed solvent thereof.

An example of the alkyl (meth)acrylate (1) to constitute the alkyl (meth)acrylate unit (I) includes n-hexyl (meth) acrylate, cyclohexyl (meth)acrylate, heptyl (meth)acrylate, octyl (meth)acrylate, 2-ethylhexyl (meth)acrylate, nonyl (meth) acrylate, decyl (meth)acrylate and lauryl (meth)acrylate. In particular, 2-ethylhexyl (meth)acrylate and lauryl (meth) acrylate are preferred in terms of a cost and performance.

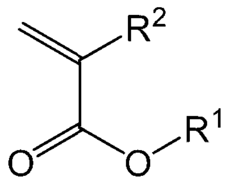

( 1 )

wherein $R^1$ is a $C_{6-20}$ alkyl group and $R^2$ a hydrogen atom or a methyl group.

The silicone (meth)acrylate having the repetition unit "m" of 1 or more and 30 or less is preferred as the silicone (meth)acrylate (2) to constitute the silicone (meth)acrylate unit (II). When the repeating unit "m" is included in the above range, the viscosity of the copolymer becomes appropriate and thus the copolymer may be kept on the surface of a medical device more surely and the copolymer can be easily treated. The repeating unit "m" is preferably 1 or more and 20 or less.

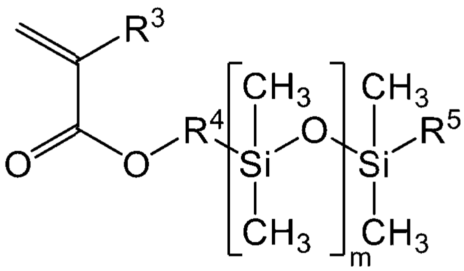

( 2 )

wherein $R^3$ is a hydrogen atom or a methyl group, $R^4$ is a $C_{1-6}$ alkylene group, $R^5$ is a $C_{1-6}$ alkyl group, m is an integer of 1 or more and 30 or less.

The ethylene oxide unit number "n" in the methoxypolyethylene glycol (meth)acrylate (3) to constitute the methoxypolyethylene glycol (meth)acrylate unit (III) is preferably 2 or more and 10 or less. When the repetition number "n" of ethylene oxide is 2 or more, the adhesion of platelet and the adsorption of a plasma protein can be suppressed more surely. When the repetition number "n" is 10 or less, the elution of the copolymer to blood can be suppressed more surely. The repetition number "n" of ethylene oxide is more preferably 2 or more and 5 or less. An example of the methoxypolyethylene glycol (meth)acrylate (3) includes methoxydiethylene glycol (meth)acrylate, methoxytriethylene glycol (meth)acrylate, methoxytetraethylene glycol (meth)acrylate, methoxypentaethylene glycol (meth)acrylate, methoxyhexaethylene glycol (meth)acrylate, methoxyheptaethylene glycol (meth)acrylate, methoxyoctaethylene glycol (meth)acrylate, methoxynonaethylene glycol (meth)acrylate and methoxydecaethylene glycol (meth)acrylate. The methoxypolyethylene glycol (meth)acrylate (3) is more preferably tetraethylene glycol(meth)acrylate having the repetition number of 4 and triethylene glycol (meth)acrylate having the repetition number of 3, and particularly preferably triethylene glycol (meth)acrylate having the repetition number of 3.

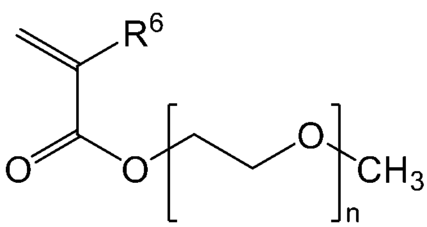

( 3 )

wherein $R^6$ is a hydrogen atom or a methyl group, n is an integer of 2 or more and 4 or less.

The proportion of the silicone (meth)acrylate unit (II) in the copolymer is preferably 50 mass% or less in comprehensive view of performance, quality and a cost. The proportion is more preferably 40 mass% or less and even more preferably 35 mass% or less. The silicone (meth)acrylate (2) and the above-described other alkyl (meth)acrylate (1) may be mixed to be used. The proportion of the silicone (meth)acrylate (2) in the hydrophobic (meth)acrylate is preferably 0.1 mass% or more. When the proportion is 0.1 mass% or more, the hydrolysis of the copolymer can be suppressed more surely and thus the stability as an antithrombotic material can be maintained more surely for a long time. The proportion is more preferably 0.5 mass% or more and even more preferably 1.0 mass% or more. The upper limit of the proportion is not particularly restricted, and the proportion may be adjusted to, for example, 50 mass% or less. The proportion is preferably 40 mass% or less or 30 mass% or less and more preferably 20 mass% or less or 10 mass% or less.

The antithrombotic material comprising the (meth)acrylate copolymer may comprise a substance such as an antibacterial substance in the present invention. An antibacterial substance can be mainly classified into a water-soluble

substance and a water-insoluble substance. An example of a water-soluble antibacterial substance representatively includes benzalkonium chloride, potassium penicillin G and streptomycin sulfate. An example of a hardly water-soluble antibacterial substance includes sulfadiazine silver and chlorhexidine. One of the substances may be used alone, or a plurality of the substances may be mixed to be used.

The ratio of an antibacterial substance to the mass of the antithrombotic composition is preferably 0.01 mass% or more and 70 mass% or less in the present invention. When the ratio is 0.01 mass% or more, the bactericidal effect of an antibacterial substance is exerted more surely. When the ratio is 70 mass% or less, the poor appearance of a medical device after a surface treatment such as coating can be inhibited more surely and the elution of an antibacterial substance to a body and local inflammatory due to an eluted antibacterial substance can be inhibited more surely. The ratio is more preferably 0.05 mass% or more and 50 mass% or less, even more preferably 0.1 mass% or more and 30 mass% or less, and particularly preferably 0.1 mass% or more and 10 mass% or less. An antibacterial substance may be present on the entire surface of a medical device, but it is preferred that an antibacterial substance is present in the vicinity only of the insertion site at which a medical device penetrates the skin in order to suppress local inflammation.

The (meth)acrylate copolymer produced by copolymerizing the alkyl (meth)acrylate (1) and/or the silicone (meth) acrylate (2) with the methoxypolyethylene glycol (meth)acrylate (3) can be preferably used as a blood-compatible material in the present invention, since the (meth)acrylate copolymer has hydrophilicity and hydrophobicity in an appropriate balance. In particular, the (meth)acrylate copolymer having the silicone (meth)acrylate unit (II) in the specific range can be preferably used as a material to treat a medical device, since the (meth)acrylate copolymer can suppress the adhesion and the adsorption of a serum protein or the like. One of the abovedescried (meth)acrylate copolymer can be used alone, or 2 or more kinds of the (meth)acrylate copolymer can be mixed to be used.

When the medical device treated with the antithrombotic material is contacted with blood, the highly hydrophilic methoxypolyethylene glycol (meth)acrylate unit (III) may bleed out through the surface to be antithrombotic, and the hydrophobic (meth)acrylate units (I) and (II) may remain near the base material and thus may prevent the medical device from directly being contacted with blood.

An aliphatic hydrocarbon solvent that can appropriately dissolve the (meth)acrylate copolymer and hardly cause damage on a medical device as a base material is selected as the aliphatic hydrocarbon solvent in the aliphatic hydrocarbon solution of the (meth)acrylate copolymer. An example of such an aliphatic hydrocarbon solvent includes a linear aliphatic hydrocarbon solvent such as n-pentane, n-hexane, n-heptane and n-octane; a cyclic aliphatic hydrocarbon solvent such as cyclopentane, methylcyclopentane, cyclohexane and methylcyclohexane; and a mixed solvent of 2 or more these solvents. The aliphatic hydrocarbon solvent is particularly preferably cyclohexane. The inventors of the present invention experimentally found that an aliphatic hydrocarbon solvent does not swell nor shrink a medical device composed of polyurethane and thus can maintain the dimensions thereof.

The reason why the dimensional change of a polyurethane medical device is reduced by using an aliphatic hydrocarbon solvent as a solvent is not well understood, but the inventors believe the reason is as follows. In Figure 1, the aliphatic hydrocarbon solvent is representatively exemplified by cyclohexane, a conventional solvent is exemplified by ethanol, and a medical device is exemplified by a tube.

When a tube as a polyurethane medical device is immersed in ethanol, a larger amount of ethanol is absorbed in a thick part of a tube and thus the internal diameter, the external diameter and the thickness of the tube may become larger as shown in Figure 1, since the solubility parameter of ethanol is relatively similar to that of polyurethane. The molecular orientation of a polyurethane resin that absorbs ethanol is resolved, and the molecular orientation of the tube becomes aligned after the ethanol is removed. The thickness may particularly become small by a drying treatment, since an apparent volume becomes small by aligning the molecular orientation. On the one hand, when a polyurethane medical device is immersed in an aliphatic hydrocarbon solvent such as cyclohexane having the solubility parameter that is relatively different from that of polyurethane, the change of the internal diameter, the external diameter and the thickness of the polyurethane medical device may be small, since the absorption rate of an aliphatic hydrocarbon solvent in the polyurethane medical device is very slow.

On the one hand, since the (meth)acrylate copolymer is highly soluble in an aliphatic hydrocarbon solvent, a uniform antithrombotic coating layer can be formed on the surface of a polyurethane medical device by using an aliphatic hydrocarbon solvent as a solvent for the (meth)acrylate copolymer solution. Thus, when an aliphatic hydrocarbon solvent is used as a solvent to coat a polyurethane medical device with the antithrombotic material containing the (meth)acrylate copolymer, the surface of the medical device can be uniformly coated with the antithrombotic material with suppressing the deformation such as the swelling and the shrinkage of the polyurethane medical device.

The concentration of the (meth)acrylate copolymer in the aliphatic hydrocarbon solution to coat the surface of a polyurethane medical device with the (meth)acrylate copolymer may be appropriately adjusted and may be adjusted to, for example, 0.01 mass% or more and 10 mass% or less. When the concentration is 1.0 mass% or more, the performance of the (meth)acrylate copolymer on the surface of a medical device may be delivered more surely. When the concentration is 0.01 mass% or more, the concentration of the coating liquid is appropriate and the handling property thereof is good. When the concentration is 10 mass% or less, the performance of the (meth)acrylate copolymer on a medical device may be

delivered more surely. The concentration is preferably 0.1 mass% or more or 0.5 mass% or more, more preferably 1.0 mass% or more or 1.5 mass% or more, even more preferably 2.0 mass% or more, and preferably 8.0 mass% or less or 5.0 mass% or less, more preferably 3.0 mass% or less or 2.5 mass% or less.

2. Applying step

A medical device is coated with the aliphatic hydrocarbon solution prepared in the above-described Step 1 in this step.

The (meth)acrylate copolymer is water-insoluble and thus can be preferably used as the main component of a surface treatment agent for a medical device in the present invention. As a specific embodiment, the surface of a base material such as a medical device is coated with a solution prepared by dissolving the antithrombotic material comprising the (meth)acrylate copolymer in an aliphatic hydrocarbon solvent, and then the aliphatic hydrocarbon solvent may be removed. An example of a method for supporting the antithrombotic material on the surface of a base material includes a publicly-known method, such as a coating method; a method utilizing graft polymerization by radiation, electron beam or ultraviolet ray; and a method utilizing the chemical reaction with the functional group of a base material. In particular, a coating method is practically preferred, since the procedure thereof in the production step is easy. A coating method is not particularly restricted, and applying method, spray method and dipping method can be used. For example, a coating method by application can be easily carried out by applying a coating liquid prepared by dissolving the antithrombotic material in an aliphatic hydrocarbon solvent on a base material, removing the excessive aliphatic hydrocarbon solvent, and then drying the base material in air. In addition, a base material after the coating is preferably dried by heating. As a result, the adhesion between the base material and the antithrombotic material becomes stronger to fix the antithrombotic material more solidly.

The method for applying the aliphatic hydrocarbon solution of the antithrombotic material comprising the (meth) acrylate copolymer on a medical device is not particularly restricted, and an ordinary method may be used. For example, the aliphatic hydrocarbon solution may be applied or sprayed on a medical device, or a medical device may be immersed in the aliphatic hydrocarbon solution. The temperature of the aliphatic hydrocarbon solution when the aliphatic hydrocarbon solution is applied on a medical device is not particularly restricted and may be adjusted to, for example, 10°C or higher and 45°C or lower. The temperature is preferably 15°C or higher and 25°C or lower and preferably room temperature or higher. The temperature may be also adjusted to 20±10°C or 20±5°C.

The amount of the aliphatic hydrocarbon solution to be applied or sprayed may be appropriately adjusted, and the amount of the (meth)acrylate copolymer to coat the surface of a medical device may be adjusted to, for example, 0.005 $mg/cm^2$ or more and 0.06 $mg/cm^2$ or less. In addition, the time to immerse a medical device in the aliphatic hydrocarbon solution may be also appropriately adjusted and may be adjusted to, for example, 1 second or more and 60 seconds or less. The time is preferably 30 seconds or less and more preferably 20 seconds or less or 15 seconds or less.

3. Drying step

In this step, the aliphatic hydrocarbon solvent is removed from the aliphatic hydrocarbon solution that is applied on the surface of the medical device in the above-described applying step 2. The method for removing the aliphatic hydrocarbon solvent is not particularly restricted. For example, natural drying, heating, reduction of pressure, blowing air and the combination thereof can be used, and blowing air is preferred due to efficiency.

The condition of blowing air may be appropriately adjusted. For example, when air is blown at experimental levels, for example, when the surface area of a medical device is lower than 10 $cm^2$, the flow rate of the air to blow may be adjusted to 1 mL/min or more and 20 L/min or less. When air is blown at industrial levels, for example, when the surface area of a medical device is 10 $cm^2$ or more, the flow rate of the air to blow may be adjusted to 10 L/min or more and 100 L/min or less. In addition, the flow rate of the air to blow may be adjusted to 10 L/min or more and 50 L/min or less in the case between the above-described cases.

The time to blow air on a medical material may be also appropriately adjusted and may be adjusted to, for example, 1 second or more and 180 seconds or less. The time is preferably 5 seconds or more or 10 seconds or more, more preferably 20 seconds or more or 25 seconds or more, and preferably 150 seconds or less or 120 seconds or less, more preferably 100 seconds or less or 50 seconds or less.

The aliphatic hydrocarbon solvent may be evaporated for natural drying by leaving the medical device on which the solution is applied to stand under an atmospheric temperature and an atmospheric pressure. The time for natural drying may be appropriately adjusted in the range that the aliphatic hydrocarbon solvent can be sufficiently removed and may be adjusted to, for example, 30 minutes or more and 24 hours or less. The condition for drying by heating may be also appropriately adjusted depending on the used aliphatic hydrocarbon solvent or the like and may be adjusted to, for example, 40°C or higher and 120°C or lower for 10 minutes or more and 10 hours or less. A pressure may be reduced for drying under reduced pressure in the range of 10 Pa or more and 100 Pa or less for 10 minutes or more and 10 hours or less.

For example, the aliphatic hydrocarbon solvent can be efficiently removed by the combination of drying by heating and drying under reduced pressure, but blowing air is preferred in terms of a cost or the like.

The (meth)acrylate copolymer tightly adheres to the medical device that is composed of polyurethane and coated with the (meth)acrylate copolymer by the present invention method due to the hydrophobic (meth)acrylate unit of the (meth)acrylate copolymer, and the activation of a blood coagulation system, the adhesion and the activation of platelet, and the activation of a complement system are suppressed due to the hydrophilic (meth)acrylate unit. Thus, the medical device that is composed of polyurethane and coated with the (meth)acrylate copolymer by the present invention method is excellent in the stability and the biocompatibility in a living body.

The medical device is not particularly restricted as long as the medical device is composed of polyurethane and is contacted to a body fluid such as blood in a living body. An example of the medical device includes a catheter such as peripheral intravenous catheter, implantable port, Huber needle, peripherally inserted central catheter, dialysis catheter, midline catheter, central intravenous catheter, cardiac catheter and transurethral catheter. In addition, an example of the medical device may include a part that may be contacted with a body fluid, such as an apex tip of a cardiac catheter, a cardiac catheter balloon and a pacemaker lead.

The present application claims the benefit of the priority date of Japanese patent application No. 2023-53423 filed on March 29, 2023. All of the contents of the Japanese patent application No. 2023-53423 filed on March 29, 2023, are incorporated by reference herein.

## EXAMPLES

The present invention is hereinafter described in detail by the examples, but is not restricted thereto.

### Example 1

#### (1) Production of (meth)acrylate copolymer

Azobisisobutyronitrile (AIBN) manufactured by FUJIFILM Wako Pure Chemical (1.2325 g) was added to methoxytriethylene glycol acrylate (MTEGA) manufactured by SHIN-NAKAMURA CHEMICAL (471.8 g), silicone methacrylate (PDMSMA) ("MCR-M11" manufactured by Gelest, 78.0 g), 2-ethylhexyl acrylate (EHA) manufactured by TOAGOSEI (694.5 g) and ethanol manufactured by KISHIDA CHEMICAL (1628.3 g) to conduct a polymerization reaction at 85°C for 3 hours. The product was dried at 85°C under atmospheric pressure for 2 hours after the polymerization reaction. Then, the product was dried at 60°C under reduced pressure for 1 hour to obtain the concentrate. The concentrate was divided into two equal parts, concentrate A and concentrate B.

Methanol manufactured by KISHIDA CHEMICAL (3162.7 g) and water (305.3 g) were added to the concentrate A (612.1 g), and the mixture was stirred for 30 minutes. The mixture was left to stand for 1.5 hours after the stirring, and the supernatant was removed by decantation. Methanol was added to the obtained precipitation, and the mixture was stirred for 30 minutes and stood still for 1.5 hours, and then the supernatant was removed. This procedure was repeated 3 times to obtain the precipitation A. The added amounts of the used methanol were 3161.7 g, 3161.4 g and 3163.9 g in sequence.

Methanol (3162.3 g) and water (303.1 g) were similarly added to the preparation B (614.4 g), and the mixture was stirred for 30 minutes. The mixture was stood still for 1.5 hours after stirring, and the supernatant was removed by decantation. Methanol was added to the obtained precipitation, and the mixture was stirred for 30 minutes and stood still for 1.5 hours, and then the supernatant was removed. This procedure was repeated 3 times to obtain the precipitation B. The added amounts of the used methanol were 3165.5 g, 3167.5 g and 3165.4 g in sequence.

The obtained precipitate A and precipitate B were put together into a whole, and the mixture was dried at 40°C under reduced pressure for 1.5 hours to obtain the copolymer 1.

#### (2) Measurement of weight average molecular weight

The copolymer 1 (15 mg) was weighed and added into a vial container, and a mobile phase for GPC measurement (3.0 mL) was added thereto. The mixture was stood still overnight. The obtained solution was filtrated using a hydrophilic PTFE membrane filter cartridge of 0.45 μm ("Millex-LH" manufactured by Nihon Millipore). For the GPC measurement, "515 HPLC pump" manufactured by Waters and "717plus Autosampler" manufactured by Waters were used, and "2×PLgel 5μMIXED-D, 7.5×300mm" manufactured by Agilent Technologies was used as a column. The column temperature was adjusted to 40°C, and tetrahydrofuran for HPLC containing dibutylhydroxytoluene as a stabilizer, manufactured by FUJIFILM Wako Pure Chemical, was used as a mobile phase. RI was used for detection, and 20 μL of a sample was injected. The molecular weight was calibrated using monodisperse polystyrene ("Easi Cal PS-1" manufactured by Agilent Technologies).

The weight average molecular weight of the copolymer 1 was 68,300.

(3) Measurement of reduced viscosity

The copolymer 1 (1 g) was weighed and added into a glass bottle, and acetone manufactured by FUJIFILM Wako Pure Chemical (15 mL) was added thereto. The mixture was shaken by hand once every 20 minutes to be mixed. It was visually confirmed after 1 hour that the copolymer was dissolved, and the mixture was transferred into a 25 mL measuring flask. The glass bottle was rinsed with acetone and the acetone was added into the measuring flask, and then acetone was further added to adjust the total amount to 25 mL. Next, the mixture was filtrated using a filter having of 5 μm (manufactured by Merck), and the filtrate was used as a test solution. The flowthrough times of the test solution and acetone were measured at 30°C using a Ubbelohde viscometer having the viscometer constant C = 0.003426 cSt/s, and the reduced viscosity was calculated by the following formula:

Reduced viscosity (nsp/c) = $(A/B-1)/C = (A/B-1)/(S/25\times100) = (A/B-1)/(S\times4)$

A: flowthrough time of test solution (sec)
B: flowthrough time of acetone (sec)
S: weight of sample (g)
C: concentration of test solution (g/dL)

The reduced viscosity of the copolymer 1 was 0.214 dl/g.

(4) Production of coating liquid

Cyclohexane (78.4 g) was added to the copolymer 1 (1.6 g), and the mixture was sufficiently mixed to produce the coating liquid 1. The concentration of the copolymer 1 in the coating liquid 1 was 2.0 mass%.

(5) Antithrombotic treatment of polyurethane tube

A polyurethane tube having the inner diameter of 2.3 mm, the outer diameter of 3.8 mm, the thickness of 0.7 mm and the length of 21 mm was immersed in the coating liquid 1 for 10 seconds, and then taken out from the coating liquid 1. The excessive cyclohexane was removed by applying air at a flow rate of 10 mL/min to each of the outer and inner surfaces for 30 seconds each. Then, the tube was dried at room temperature for 16 hours to obtain the polyurethane tube 1 of which surface was coated with the (meth)acrylate copolymer as an antithrombotic material.

(6) Measurement of surface adhesion rate of (meth)acrylate copolymer

The copolymer 1 (20 mg) was dissolved in chloroform-d (1 mL) to analyze the copolymer at 30°C by $^1$H-NMR. The A to D were calculated by the following calculation formulas on the basis of the obtained peak areas:

A: peak integral value of one methoxy group of MTEGA around 3.4 ppm / 3 (number of H) × 218 g/mol (molecular weight of MTEGA)
B: peak integral value of two methyl groups of EHA around 0.8 ppm / 6 (number of H) × 184 g/mol (molecular weight of EHA)
C: peak integral value of two methylene groups of PDMSMA around 0.5 ppm / 4 (number of H) × 971 g/mol (molecular weight of PDMSMA)
D: integral value of peak derived from PDMSMA around 0 ppm Then, the coated polyurethane tube was immersed in ethanol (5 mL) for 30 minutes to obtain an extraction liquid. The obtained extraction liquid was dried by blowing nitrogen gas at 40°C until ethanol odor became imperceptible. This procedure was repeated 3 times. Dimethyl isophthalate (DMI) was added to the whole dried extract, and then the mixture was dissolved in chloroform-d (0.6 mL) to be analyzed at 30°C by $^1$H-NMR. The E and F were calculated by the following calculation formulas on the basis of the obtained peak areas:
E: integral value of peak derived from a benzene group of DMI around 7.5 ppm / 1 (number of H) × 194 g/mol (molecular weight of DMI)
F: integral value of peak derived from PDMSMA around 0 ppm
G: surface area of polyurethane tube ($cm^2$)

The surface adhesion amount was calculated by the following calculation formula on the basis of the above-described A to G.

Surface adhesion amount (mg/cm$^2$) = [F×{(A+B+C)/D}]×(additive amount of DMI (mg)/E)/G

Table 1

| Coating solvent | Concentration of (meth)acrylate copolymer in coating liquid | Surface area | Surface adhesion amount |
|---|---|---|---|
| cyclohexane | 2.0 mass% | 1.58 cm$^2$ | 0.0258 mg/cm$^2$ |

It is found from the result shown in Table 1 that the surface of a polyurethane tube can be uniformly coated with the antithrombotic material by using cyclohexane as a coating solvent.

(7) Measurement of inner diameter

The polyurethane tube was stood on the stage of an optical microscope ("Digital microscope VHX-900" manufactured by KEYENCE; "VH - 100R" or "VH - Z20R" was used as a lens) to measure the inner diameter and the outer diameter at one side. The inner diameters and the outer diameters of 5 polyurethane tubes were measured at arbitrary 3 points in one cross-section of the polyurethane tubes by the diameter menu of a software, and the average values thereof were calculated.

(8) Calculation of change rates of inner diameter, outer diameter and thickness

The change rates of the inner diameter, the outer diameter and the thickness of the polyurethane tube were calculated by the following formula:

Change rate (%) = [(measured value after treatment - measured value before treatment) / measured value before treatment]×100

Comparative example 1

The polyurethane tube 2 was produced by coating a polyurethane tube with the (meth)acrylate copolymer similarly to Example 1 except that ethanol was used in place of cyclohexane.

Test example 1: Effect by solvent

A polyurethane tube was immersed in the coating liquid for 10 seconds only to be coated in Example 1 and Comparative example 1. On the one hand, the effect by a solvent was tested by immersing a polyurethane tube in each solvent for longer time.

Specifically, the produced tube 1 was immersed in cyclohexane for 23 hours and then dried at about 20°C for 5 hours. The thickness, the inner diameter and the outer diameter of the tube were measured before the immersion treatment, 15 seconds, 1 hour, 2 hours, 3 hours, 4 hours, 5 hours, 21 hours and 23 hours after the immersion treatment, and 1 hour, 2 hours, 3 hours, 4 hours and 5 hours after the drying treatment. The measurement result is shown in Table 2 and Figures 2 to 4.

Table 2

| Step | Treating time | Average inner diameter (n=5) | Inner diameter change rate | Average outer diameter (n=5) | Outer diameter change rate | Average thickness (n=5) | Thickness diameter change rate |
|---|---|---|---|---|---|---|---|
| Immersion in cyclohexane | Before treatment | 2339μm | - | 3814μm | - | 737μm | - |
| | 0.004hr | 2346μm | 0.3% | 3800μm | -0.4% | 727μm | -1.4% |
| | 1hr | 2369μm | 1.3% | 3834μm | 0.5% | 733μm | -0.7% |
| | 2hr | 2366um | 1.1% | 3839um | 0.7% | 737μm | -0.1% |
| | 3hr | 2391um | 2.2% | 3848um | 0.9% | 729μm | -1.2% |
| | 4hr | 2381μm | 1.8% | 3877μm | 1.7% | 748μm | 1.5% |
| | 5hr | 2397μm | 2.5% | 3884μm | 1.8% | 743μm | 0.8% |
| | 21hr | 2429μm | 3.8% | 3919μm | 2.8% | 745μm | 1.0 |
| | 23hr | 2456μm | 5.0% | 3944μm | 3.4% | 744μm | 0.8% |
| Drying treatment | 1hr | 2417μm | 3.3% | 3885μm | 1.9% | 734μm | -0.5% |
| | 2hr | 2394μm | 2.3% | 3869%μm | 1. 4% | 738μm | 0.0% |
| | 3hr | 2389um | 2.1% | 3858um | 1.2% | 735um | -0.4% |
| | 4hr | 2385μm | 1.9% | 3827μm | 0.3% | 721μm | -2.2% |
| | 5hr | 2375μm | 1.5% | 3826μm | 0.3% | 725pm | -1.7% |

The inner diameter, the outer diameter and the thickness of the tube were similarly measured except that ethanol was used in place of cyclohexane for comparison. The measurement result is shown in Table 3 and Figures 2 to 4.

Table 3

| Step | Treating time | Average inner diameter (n=5) | Inner diameter change rate | Average outer diameter (n=5) | Outer diameter change rate | Average thickness (n=5) | Thickness diameter change rate |
|---|---|---|---|---|---|---|---|
| Immersion in ethanol | Before treatment | 2341μm | - | 3793μm | - | 726μm | - |
| | 0.004hr | 2365μm | 1.0% | 3808μm | 0.4% | 721μm | -0.6% |
| | 1hr | 2548μm | 8.8% | 4077μm | 7.5% | 765μm | 5.3% |
| | 2hr | 2683μm | 14.6% | 4248μm | 12.0% | 782μm | 7.8% |
| | 3hr | 2695um | 15.1% | 4254um | 12.2% | 779μm | 7.3% |
| | 4hr | 2768μm | 18.3% | 4323um | 14.0% | 777μm | 7.1% |
| | 5hr | 2755μm | 17.70 | 4310um | 13.6% | 778μm | 7.1% |
| | 21hr | 2755um | 17.7% | 4294μm | 13.20 | 769μm | 5.9% |
| | 23hr | 2759μm | 17.8% | 4281μm | 12.8% | 761μm | 4.8% |
| Drying treatment | 1hr | 2473μm | 5.6% | 3915μm | 3.2% | 721μm | -0.7% |
| | 2hr | 2414μm | 3.1% | 3869μm | 2.0% | 727μm | 0.2% |
| | 3hr | 2425μm | 3.6% | 3841um | 1.3% | 708μm | -2.5% |
| | 4hr | 2399μm | 2.5% | 3822um | 0.8% | 712μm | -2.0% |
| | 5hr | 2420um | 3.4% | 3828μm | 0.9% | 704μm | -3.1% |

It was confirmed from the results shown in Tables 2 and 3 and Figures 2 to 4 that the swelling and the shrinkage of the inner diameter, the outer diameter and the thickness were suppressed to be smaller in the Example prepared by

coating a polyurethane tube with the (meth)acrylate copolymer with using cyclohexane in comparison with the Comparative example coated with using ethanol.

Test example 2: Blood compatibility test

A polyurethane tube having the inner diameter of 0.82 mm, the outer diameter of 1.12 mm and the length of 21 mm was immersed in the coating liquid 1 for 10 seconds, and then taken out. The excessive cyclohexane was removed by applying air at a flow rate of 10 mL/min to each of the outer and inner surfaces of the tube for 30 seconds each. Then, the tube was dried at room temperature for 16 hours to fix the antithrombotic material on the surface of the tube.

The obtained tube was cut into 1 cm length and added into a 15 mL centrifuge tube. Separately, test blood was prepared by mixing blood of a rabbit and 80 mM calcium chloride normal saline solution in ratio of 12:1 by volume. The test blood (1 mL) was added into a centrifuge tube and incubated at 37°C for 25 minutes. Then, the tube was taken out from the centrifuge tube and transferred to the centrifuge tube containing normal saline solution (5 mL) to be shaken using "VORTEX SI-0246" manufactured by Scientific Industries with shaking degree setup = 6 for 5 seconds. The tube was taken out from the normal saline solution, and the conditions of blood clots adhered on the outer surface and the inner cavity were visually observed. The above test was conducted using 5 tubes. The result is shown in Figure 5.

It is found from the result shown in Figure 5 that the surface of a polyurethane tube can be coated with the antithrombotic material by using cyclohexane as a coating solvent, since the formation of blood clot and thrombus were not observed at any tubes.

INDUSTRIAL APPLICABILITY

A high quality antithrombotic polyurethane medical device can be provided according to the present invention, since not only at least a part of the body fluid contact part of a polyurethane medical device can be uniformly coated with the antithrombotic material but also the swelling and the shrinkage of a medical device due to a coating treatment can be suppressed.

**Claims**

1. A method for producing an antithrombotic medical device, comprising the steps of:

   dissolving an antithrombotic material comprising a (meth)acrylate copolymer in an aliphatic hydrocarbon solvent to prepare an aliphatic hydrocarbon solution,
   coating a medical device with the aliphatic hydrocarbon solution, and
   removing the aliphatic hydrocarbon solvent from a surface of the medical device,
   wherein the medical device is composed of polyurethane.

2. The method according to claim 1, wherein a concentration of the (meth)acrylate copolymer in the aliphatic hydrocarbon solution is 0.01 mass% or more and 10 mass% or less.

3. The method according to claim 1, wherein a temperature of the aliphatic hydrocarbon solution is 10°C or higher and 45°C or lower.

4. The method according to claim 1, wherein the aliphatic hydrocarbon solvent is removed by blowing air on the medical device in a flow rate of 1 mL/min or more and 100 L/min or less.

5. The method according to claim 4, wherein the air is blown on the medical device for 1 second or more and 180 seconds or less.

6. The method according to claim 1, wherein the (meth)acrylate copolymer comprises an alkyl (meth)acrylate unit represented by the following formula (I), a silicone (meth)acrylate unit represented by the following formula (II) and a methoxypolyethylene glycol (meth)acrylate unit represented by the following formula (III):

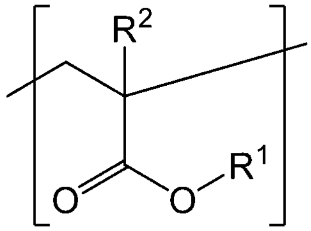

(I)

wherein $R^1$ is a $C_{6-20}$ alkyl group, and $R^2$ is a hydrogen atom or a methyl group,

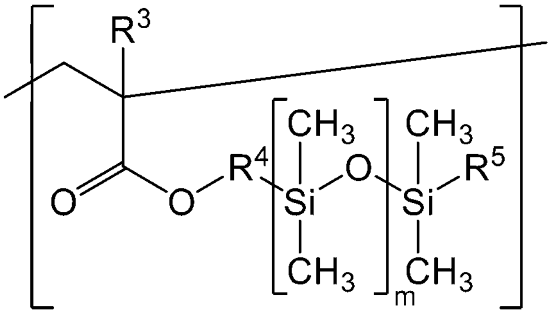

(II)

wherein $R^3$ is a hydrogen atom or a methyl group, $R^4$ is a $C_{1-6}$ alkylene group, $R^5$ is a $C_{1-6}$ alkyl group, and m is an integer of 1 or more and 30 or less,

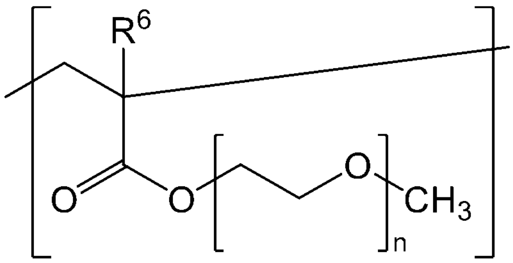

(III)

wherein $R^6$ is a hydrogen atom or a methyl group, and n is an integer of 2 or more and 4 or less.

**7.** The method according to claim 6, wherein a molar ratio of the alkyl (meth)acrylate unit/the silicone (meth)acrylate unit/the methoxypolyethylene glycol (meth)acrylate unit in the (meth)acrylate copolymer is 80-20/10-0.01/10-79.99.

**8.** The method according to claim 1, wherein a weight average molecular weight of the (meth)acrylate copolymer is 50,000 or more and 1,500,000 or less.

**9.** The method according to claim 1, wherein a reduced viscosity of the (meth)acrylate copolymer is 0.18 dl/g or more and 0.50 dl/g or less.

**10.** The method according to claim 1, wherein the medical device is coated with 0.005 mg/$cm^2$ or more and 0.06 mg/$cm^2$ or less of the (meth)acrylate copolymer.

**11.** A medical device,

composed of polyurethane, and
coated with 0.005 mg/$cm^2$ or more and 0.06 mg/$cm^2$ or less of a (meth)acrylate copolymer.

[Fig. 1]

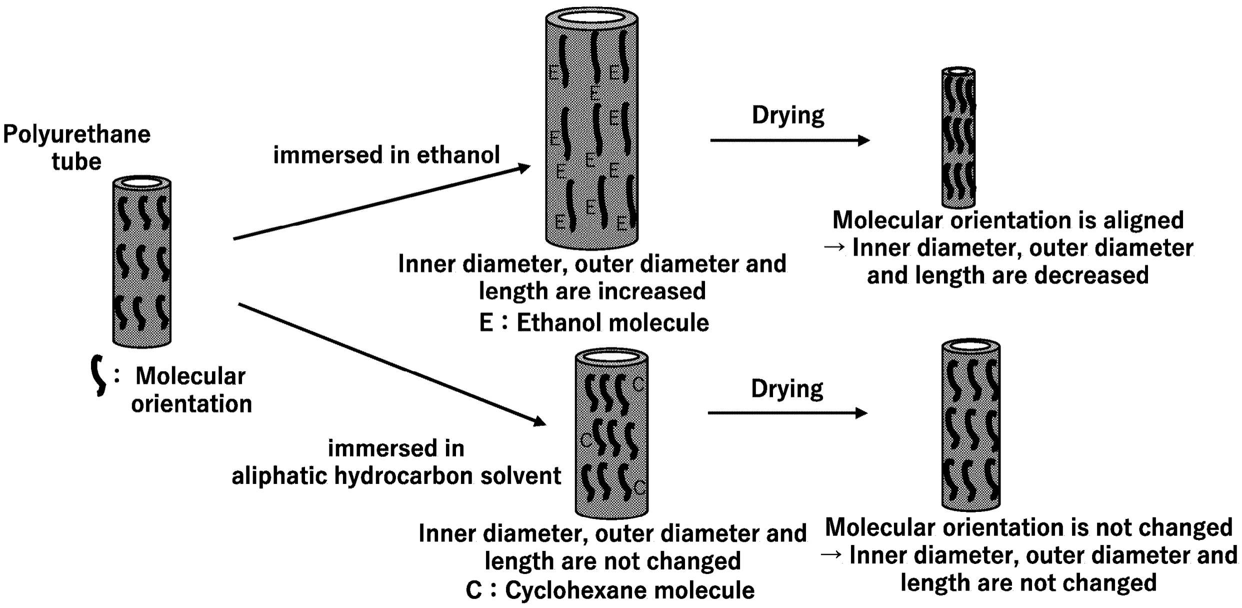
Polyurethane tube
: Molecular orientation
immersed in ethanol
Inner diameter, outer diameter and length are increased
E : Ethanol molecule
Drying
Molecular orientation is aligned
→ Inner diameter, outer diameter and length are decreased
immersed in aliphatic hydrocarbon solvent
Inner diameter, outer diameter and length are not changed
C : Cyclohexane molecule
Drying
Molecular orientation is not changed
→ Inner diameter, outer diameter and length are not changed

[Fig. 2]

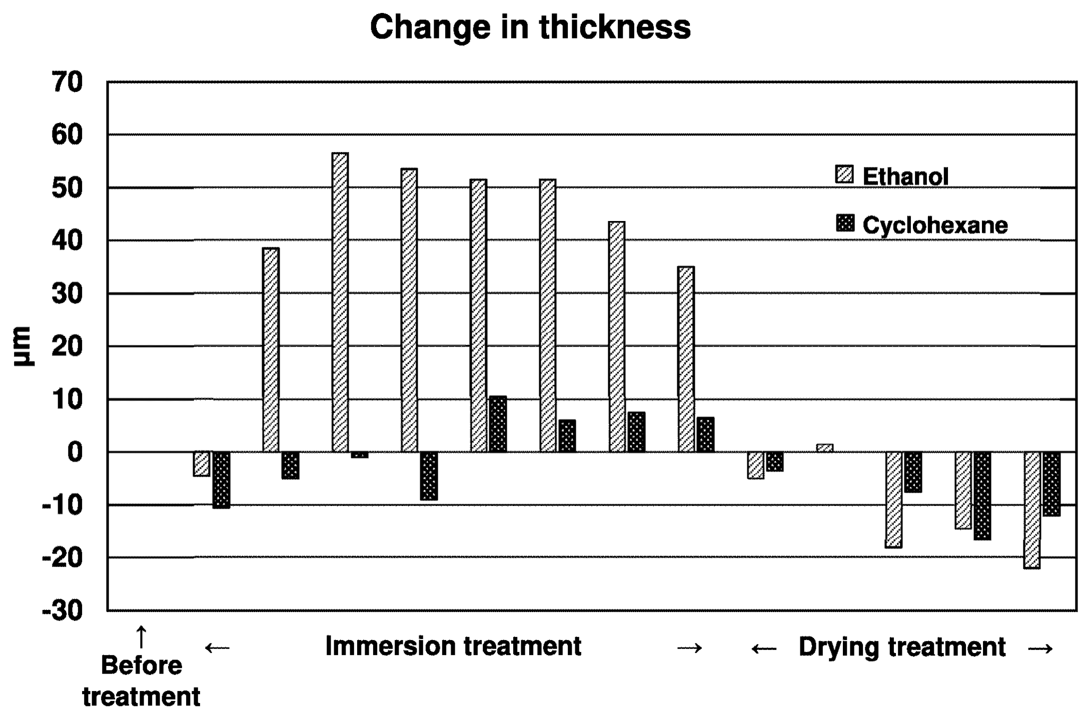
Change in thickness
70
60
50
40
30
20
10
0
-10
-20
-30
μm
Ethanol
Cyclohexane
↑
Before
treatment
← Immersion treatment →
← Drying treatment →

[Fig. 3]

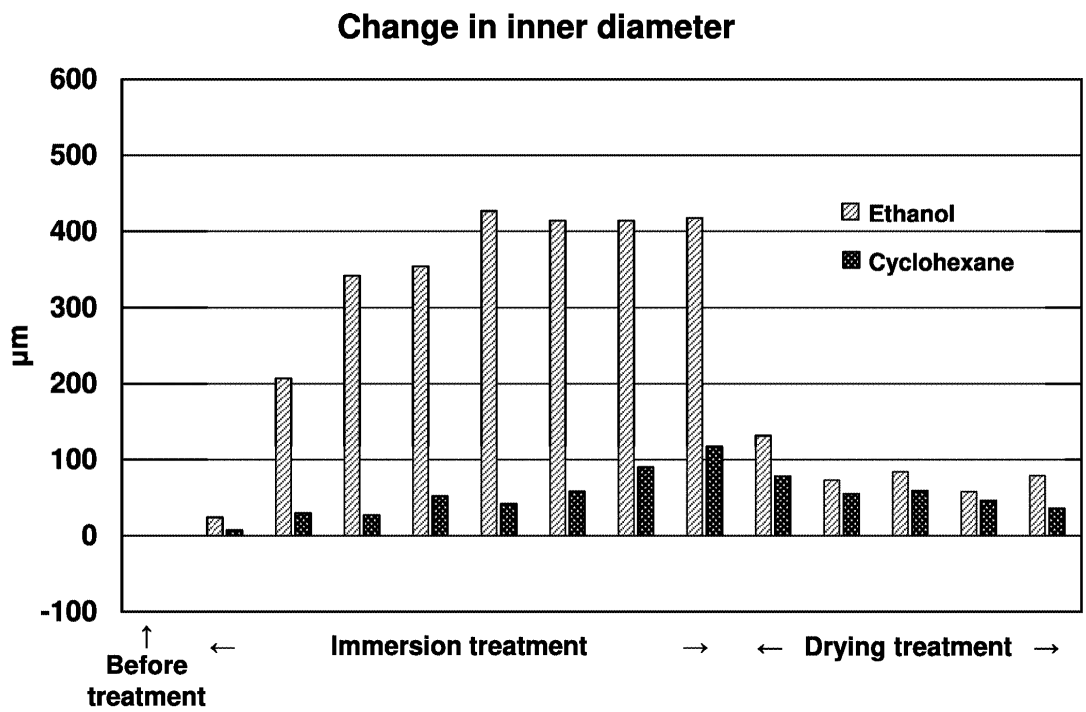
Change in inner diameter
600
500
400
300
200
100
0
-100
μm
Ethanol
Cyclohexane
↑
Before
treatment
← Immersion treatment →
← Drying treatment →

[Fig. 4]

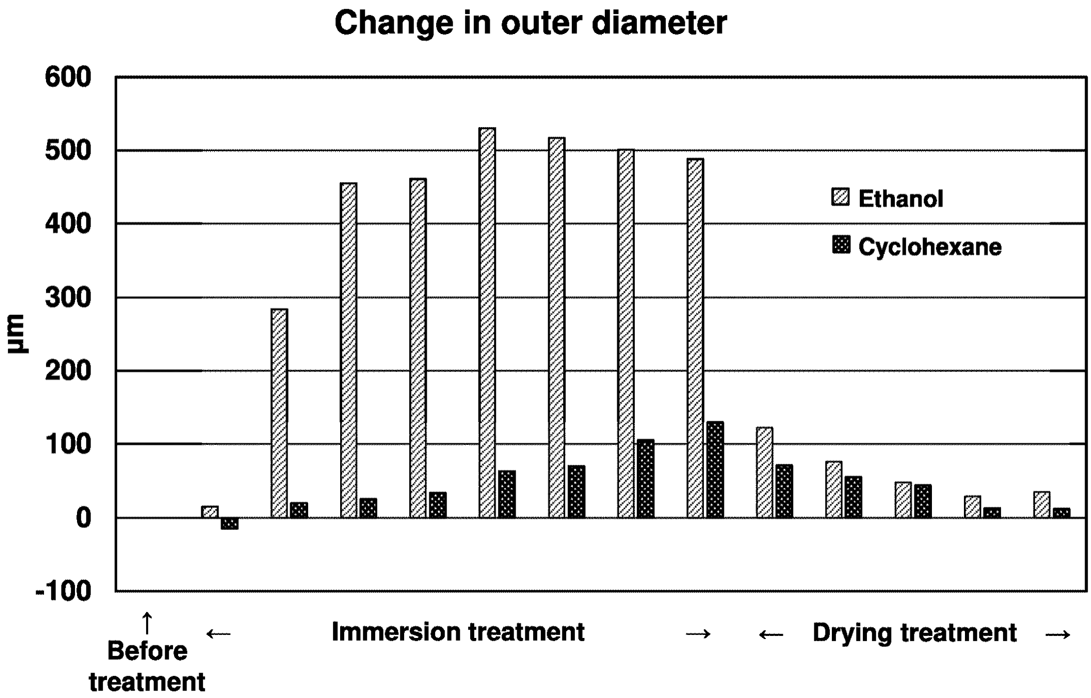
Change in outer diameter
600
500
400
300
200
100
0
-100
µm
Ethanol
Cyclohexane
↑
Before
treatment
← Immersion treatment →
← Drying treatment →

[Fig. 5]

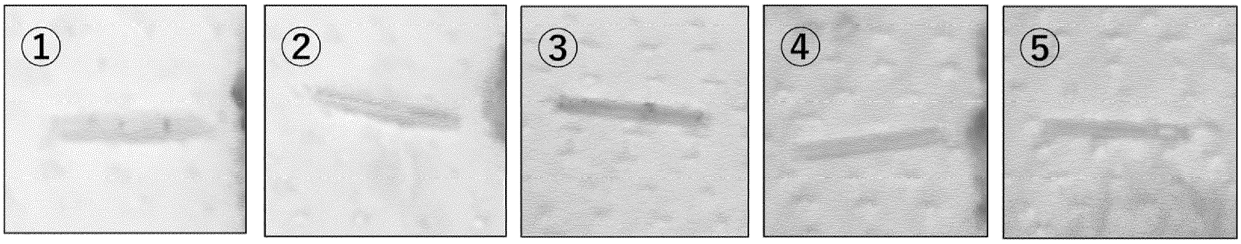

## INTERNATIONAL SEARCH REPORT

International application No.
**PCT/JP2024/011857**

**A. CLASSIFICATION OF SUBJECT MATTER**

***A61L 33/06***(2006.01)i; ***A61L 29/06***(2006.01)i
FI: A61L33/06 200; A61L29/06

According to International Patent Classification (IPC) or to both national classification and IPC

**B. FIELDS SEARCHED**

Minimum documentation searched (classification system followed by classification symbols)

A61L29/00-29/18; A61L31/00-31/18; A61L33/00-33/18

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 2009-261437 A (TOYO BOSEKI KABUSHIKI KAISHA) 12 November 2009 (2009-11-12)<br>claims 2, 4, paragraphs [0016]-[0022], [0026]-[0033], [0042], [0044], [0045], [0089], [0103], [0108], [0114], [0123] | 1-11 |
| Y | JP 2007-168310 A (ITO KOGAKU KOGYO K.K.) 05 July 2007 (2007-07-05)<br>paragraph [0055] | 1-11 |
| Y | JP 2019-136204 A (SAN MEDICAL GIJUTSU KENKYUSHO K.K.) 22 August 2019 (2019-08-22)<br>paragraphs [0063], [0073] | 1-11 |
| Y | JP 2019-155577 A (FUJIBO HOLDINGS, INC.) 19 September 2019 (2019-09-19)<br>paragraph [0012] | 1-11 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 May 2024** | **11 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/011857**

**C. DOCUMENTS CONSIDERED TO BE RELEVANT**

| Category* | Citation of document, with indication, where appropriate, of the relevant passages | Relevant to claim No. |
|---|---|---|
| Y | 林安男. プラスチックと他の材料. 日本ゴム協会誌. 1972, vol. 45, no. 11, pp. 999-1010, (Journal of the Society of Rubber Science and Technology, Japan), non-official translation (HAYASHI, Yasuo. Plastics and Other Materials.) p. 1003, left column, lines 27-37, p. 1004, table 5 | 1-11 |

Form PCT/ISA/210 (second sheet) (July 2022)

**INTERNATIONAL SEARCH REPORT**
**Information on patent family members**

International application No.
**PCT/JP2024/011857**

| Patent document cited in search report | Publication date (day/month/year) | Patent family member(s) | Publication date (day/month/year) |
|---|---|---|---|
| JP 2009-261437 A | 12 November 2009 | (Family: none) | |
| JP 2007-168310 A | 05 July 2007 | (Family: none) | |
| JP 2019-136204 A | 22 August 2019 | (Family: none) | |
| JP 2019-155577 A | 19 September 2019 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4100452 B
- JP 2009261437 A
- JP 2023053423 A